# EUROPEAN PATENT APPLICATION

(11) **EP 4 375 266 A1**
(43) Date of publication of application: **29.05.2024**
(21) Application number: 22306742.2
(22) Date of filing: 25.11.2022
(51) Int. Cl.: C07C 45/36, B01J 31/00

(54) **OXIDATION OF ALKENE INTO KETONE**

(71) Applicant: Centre National de la Recherche Scientifique, 75016 Paris (FR); Université de Rennes, 35042 Rennes (FR); École Nationale Supérieure de Chimie, 35700 Rennes (FR); Institut National des Sciences Appliquées de Rennes, 35700 Rennes (FR)
(72) Inventor: GRAMAGE-DORIA, Rafael, 75016 Paris (FR); TROUVE, Jonathan, 75016 Paris (FR); YOUSSEF, Khalil, 75016 paris (FR); KASEMTHAVEECHOK, Sitthichok, 75016 Paris (FR)
(74) Representative: Icosa

(57) **Abstract**

The present invention relates to the use of a compound of formula (I) as a catalyst for the oxidation of an alkene into a ketone and to a process of oxidation of an alkene into a ketone comprising a step (a) of reacting said alkene with an oxidant in the presence of a catalyst of formula (I).

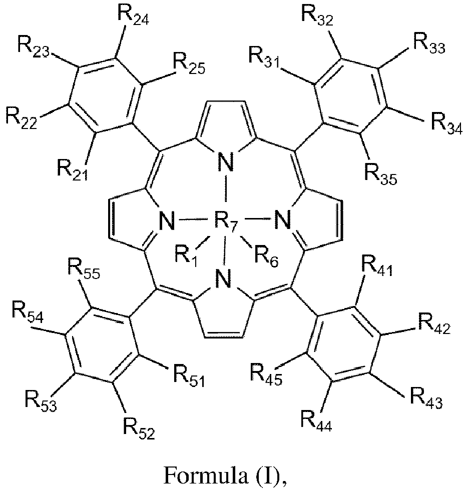

## Description

### FIELD OF INVENTION

The present invention relates to the use of a compound of formula (I) as a catalyst for the oxidation of an alkene into a ketone and to a process of oxidation of an alkene into a ketone.

### BACKGROUND OF INVENTION

Ketones are of great interest to industry. They are used, for example, as solvents in fine chemistry, as colorants in perfumery and for the manufacture of various plastics.

A protocol to produce ketones from the oxidation of olefins, which is widely used, involves the use of a palladium catalyst. However, this protocol requires specific conditions: high oxygen pressure, high temperature, acidic environment, and the palladium catalyst is expensive and scarce.

Ketones can also be synthesized by the Hock process, which involves the use of cumene (obtained from benzene and propylene) to produce acetone and acetophenone compounds, this after multiple distillation and purification steps. This process also requires specific conditions, especially because some of the compounds used, such as benzenes, are carcinogenic.

In order to develop simpler and more environmentally friendly protocols, researchers have recently been interested in iron-based catalysts. Work reported by Han *et al.* has "softened" the reaction conditions by using an inorganic iron salt in the presence of a polymethylhydrosiloxane reductant operating in ambient air and by using a solvent such as ethanol (B. Liu et al., Angew. Chem. Int. Ed. 2017, 56, 12712-12717). More recently, Knoelker and his collaborators have developed a protocol still using an iron-based phthalocyanine catalyst under reduced load and operating at room temperature but under oxygen pressure (1 bar) (F. Puls et al., Angew. Chem. Int. Ed. 2018, 57, 1222-1226 and Puls et al., Chem. Eur. J. 2021, 27, 16776-16787). A recent protocol from the same group operates under ambient pressure (Puls et al., Angew. Chem. Int. Ed. 2021, 60, 14083-14090). The protocols implemented by the teams of Han and Knoelker, independently, produced interesting quantities of ketones but these productions were accompanied by the significant production of additional alcohols as by-products.

The development of olefin oxidation protocols to selectively produce ketones under "mild" and more environmentally friendly conditions remains a technical challenge.

The inventors of the present invention have surprisingly discovered that the use of a compound of formula (I) as a catalyst for the oxidation of an alkene into a ketone allows said oxidation to be carried out under mild conditions, without the use of cancerogenic compounds such as isoprene, benzene or cumene, and with a high selectivity for the production of ketones.

### SUMMARY

The present invention relates to the use of a compound of formula (I) as a catalyst for the oxidation of an alkene into a ketone, wherein formula (I) is as follows: 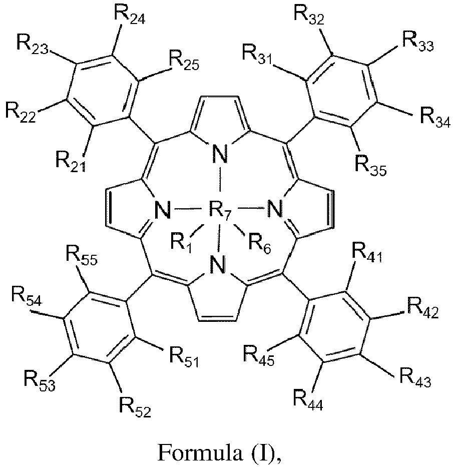 wherein R₆, which may be present or absent, is an optional group -Z, wherein group -Z is as follows: 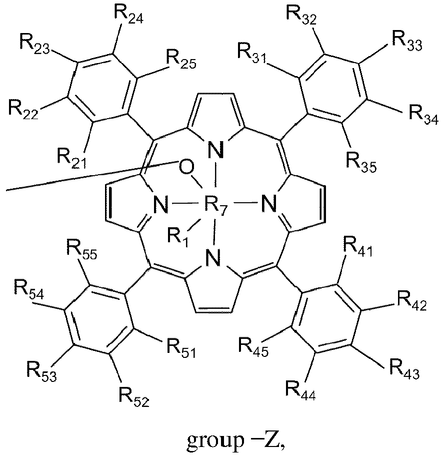 wherein, for formula (I) and for group -Z:
- R₁, which may be present or absent, is an optional anion selected from the group consisting of: Cl⁻, Br⁻, HO⁻ and CH₃COO⁻ ;
- R₂₁, R₂₂, R₂₃, R₂₄, R₂₅, R₃₁, R₃₂, R₃₃, R₃₄, R₃₅, R₄₁, R₄₂, R₄₃, R₄₄, R₄₅, R₅₁, R₅₂, R₅₃, R₅₄ and R₅₅ are selected, independently from each other, from the group consisting of: -H, -F, -CO₂H, -SO₃H and -H₂PO₃; and
- R₇ is selected from the group consisting of: -Fe and -Ni,
wherein, in formula (I): when R₇ is -Ni: R₆ is always absent, and R₁ is present if R₇ is -Ni(III) and R₁ is absent if R₇ is -Ni(II).

Advantageously, R₂₁, R₂₂, R₂₃, R₂₄, R₂₅, R₃₁, R₃₂, R₃₃, R₃₄, R₃₅, R₄₁, R₄₂, R₄₃, R₄₄, R₄₅, R₅₁, R₅₂, R₅₃, R₅₄ and R₅₅ are each -F.

Advantageously, the compound of formula (I) has the following formula (Ia3): 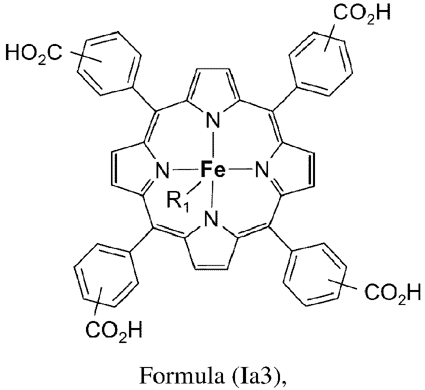 wherein R₁ is as defined above. More advantageously, the compound of formula (I) has the following formula (Ib): 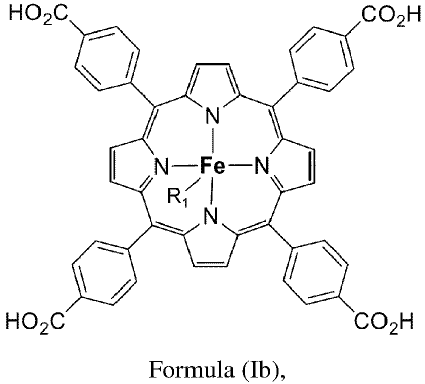 wherein R₁ is as defined above.

In one embodiment, the compound of formula (I) has the following formula (Ie):

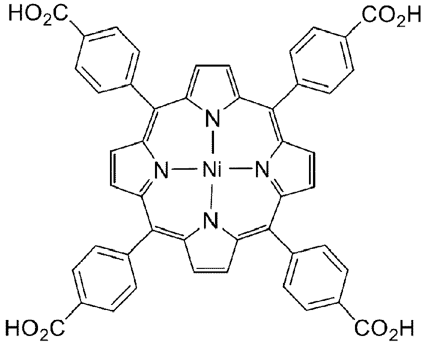

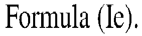

In another embodiment, the compound of formula (I) has the following formula (If):

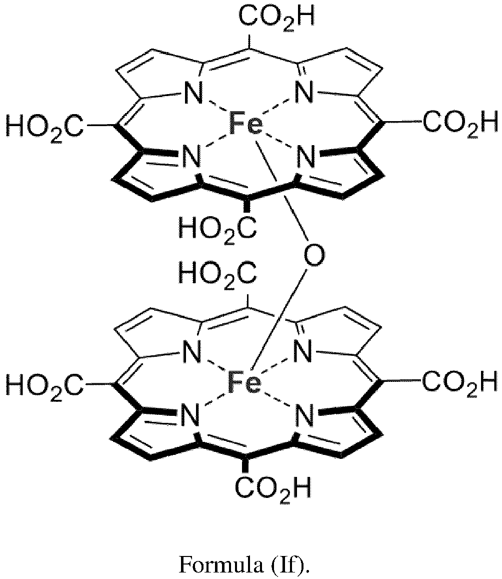

Advantageously, R₁ is Cl⁻.

The present invention also relates to a process of oxidation of an alkene into a ketone comprising a step (a) of reacting said alkene with an oxidant in the presence of a catalyst, wherein said catalyst is a compound of formula (I) as defined above.

Advantageously, step (a) is carried out at a temperature ranging from 0°C to 100°C, preferably at a temperature ranging from 0°C to 50°C, more preferably at a temperature ranging from 15°C to 30°C, even more preferably at a temperature ranging from 20°C to 25°C, better at a temperature of about 20°C.

Advantageously, step (a) is carried out at atmospheric pressure and/or at air atmosphere.

Advantageously, step (a) is carried out in the presence of at least one compound selected from the group consisting of: 1,1,3,3-tetramethyldisiloxane and compounds of formula R_{4-y}SiH_{y} (II), wherein y is an integer ranging from 1 to 3 and R is selected from the group consisting of: a C₁-C₆ alkyl and an aryl, preferably R is either an ethyl or a phenyl. More advantageously, step (a) is carried out in the presence of at least one compound selected from the group consisting of: 1,1,3,3-tetramethyldisiloxane, Et₃SiH, Ph₃SiH, Ph₂SiH₂ and PhSiH₃. Even more advantageously, step (a) is carried out in the presence of at least one compound selected from the group consisting of: Et₃SiH, Ph₃SiH, Ph₂SiH₂ and PhSiH₃.

Advantageously, step (a) is carried out in the presence of at least one compound of formula R_{4-y}SiH_{y} (II), wherein y is an integer ranging from 1 to 3 and R is selected from the group consisting of: a C₁-C₆ alkyl and an aryl, preferably R is either an ethyl or a phenyl. More advantageously, step (a) is carried out in the presence of at least one compound selected from the group consisting of: Et₃SiH, Ph₃SiH, Ph₂SiH₂ and PhSiH₃.

Advantageously, step (a) is carried out in the presence of a polymethylhydrosiloxane, preferably in the presence of a polymethylhydrosiloxane having a weight average molar mass ranging from 5,000 g.mol⁻¹ to 400,000 g.mol⁻¹.

Advantageously, the alkene is an aromatic alkene. Preferably, said alkene is selected from the group consisting of styrene, tert-butylstyrene, 3-methyl-1*H*-indole, 4-methoxystyrene, 4-chlorostyrene, 4-bromostyrene, 4-trifluoromethyl-styrene, 4-methoxycarbonylstyrene, 4-carboxystyrene, 2,4-dimethylstyrene, 4-vinylpyridine and mixtures thereof. More preferably, said alkene is styrene.

Advantageously, the oxidant is selected from the group consisting of O₂, H₂O₂, and potassium peroxymonosulfate. Preferably, the oxidant is O₂.

Advantageously, the alkene and the catalyst are in the same phase or in different phases during step (a). Preferably, the alkene and the catalyst are in the same phase during step (a).

Advantageously, step (a) is carried out in a homogeneous liquid medium comprising a solvent comprising at least 1% v/v of water. Preferably, step (a) is carried out in a homogeneous liquid medium comprising an alcohol-based solvent comprising at least 1% v/v of water. More preferably, step (a) is carried out in a homogeneous liquid medium comprising an alcohol-based solvent comprising from 2% v/v to 10% v/v of water. Even more preferably, step (a) is carried out in a homogeneous liquid medium comprising ethanol as solvent, said ethanol comprising about 4% v/v of water.

Advantageously, the step (a) is followed by a step (b) of recovering the compound of formula (I). Preferably, the step (a) is followed by a step (b) of recovering the compound of formula (I) by acid/base precipitation.

### DEFINITIONS

In the present invention, the following terms have the following meanings:
"About", before a figure or number, refers to plus or minus 10% of the face value of that figure or number. In one embodiment, "about", before a figure or number, refers to plus or minus 5% of the face value of that figure or number.

**"Acetophenone"** refers to the molecule of the following formula:

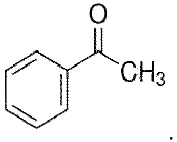

**"Alkanes"** refers to linear or cyclic, branched or unbranched, hydrocarbons having the general formula C*ₙ*H_{2*n*+2}.

**"Alkyl"** refers to carbon-centered radicals of formula -C*ₙ*H_{2*n*+1} which are derived by removal of one hydrogen atom from an alkane.

**"Alkene"** or **"olefin"** refers to linear or cyclic, branched or unbranched, hydrocarbons having at least one carbon-carbon double bond and the general formula C*ₙ*H_{2*n*}. **"Aromatic alkenes"** refers to alkenes meeting the definition of an aromatic compound. **"Aliphatic alkenes"** refers to alkenes which are not aromatic alkenes.

**"Anion"** refers to a monoatomic or polyatomic species having one or more elementary charges of the electron.

**"Aromatic compound"** refers to a compound meeting the Hückel's rule according to which a planar ring molecule has aromatic properties if it has 4n + 2 π electrons, where n is a non-negative integer.

**"Aryl"** refers to a group derived from arenes by removal of a hydrogen atom from a ring carbon atom. **"Arene"** refers to monocyclic and polycyclic aromatic hydrocarbons.

**"Atmospheric pressure"** refers to a pressure of about 1 013.25 hPa (=1 atm).

**"Catalysis"** refers to a reaction during which a catalyst is used. Catalysis can be classified as homogeneous catalysis, in which only one phase is involved, and heterogeneous catalysis, in which the reaction occurs at or near an interface between at least two phases. **"Homogeneous catalysis"** refers to a catalysis in which the reagents are in the same phase as those of the catalyst. **"Heterogeneous catalysis"** refers to a catalysis in which the reagents are not in the same phase as those of the catalyst.

**"Catalyst"** refers to a substance that increases the rate of a reaction without modifying the overall standard Gibbs energy change in the reaction and that is returned intact at the end of the reaction. According to the present invention, a catalyst has a turn-over number of strictly greater than 1 (i.e. at least 2).

**"Comprising"** or **"comprise"** is to be construed in an open, inclusive sense, but not limited to the features following this term.

**"Consisting of**" or **"consist"** is to be construed in a close, non-inclusive sense, limited to the features following this term.

**"From X to Y"** refers to the range of values between X and Y, the limits X and Y being included in said range.

**"High selectivity"** refers to a selectivity of at least 95% w/w, preferably at least 98% w/w, more preferably at least 99% w/w, even more preferably at least 99.9% w/w, for the synthesis of a product, with regards to the total weight of the products synthesized.

**"Ketone"** refers to a compound in which a carbonyl group is bonded to two carbon atoms, a ketone has the formula: R₂C=O (neither R may be H).

**"Oxidant"** or **"oxidizing agent"** refers to a substance which, in a redox chemical reaction, gains at least one electron.

**"Oxidation"** refers to the complete, net removal of one or more electrons from a molecule. During the oxidation of an alkene into a ketone, there is a gain of oxygen.

**"Phase"** refers to the state of a matter, which can be selected from the group consisting of: gas, liquid, solid and plasma.

**"Polymethylhydrosiloxane"** or **"PMHS"** refers to a polymer with the general formula -(CH₃(H)Si-O)-.

**"Precipitation"** refers to the sedimentation of a solid material (a precipitate) from a liquid solution in which the solid material is present in amounts greater than its solubility in the liquid. **"Acid/base precipitation"** refers to a precipitation wherein there is an acid-base couple in the liquid solution and only the acid or only the base is able to precipitate.

**"Room temperature"** or **"rt"** refers to a temperature ranging from 20°C to 25°C, preferably a temperature of 20°C.

**"Styrene"** refers to the molecule of the following formula:

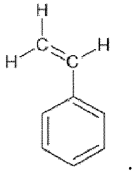

**"1,1,3,3-tetramethyldisiloxane"** refers to the molecule of the following formula:

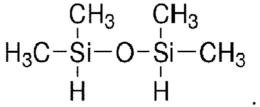

**"Turn-over number"** or **"TON"** refers to the number of moles of substrate that a mole of catalyst can convert before becoming inactivated.

**"Turn-over frequency"** or **"TOF"** refers to the turnover number per unit time.

### DETAILED DESCRIPTION

### Use of a compound of formula (I) as a catalyst for the oxidation of an alkene into a ketone

The present invention relates to the use of a compound of formula (I) as a catalyst for the oxidation of an alkene into a ketone.

Said formula (I) is as follows: 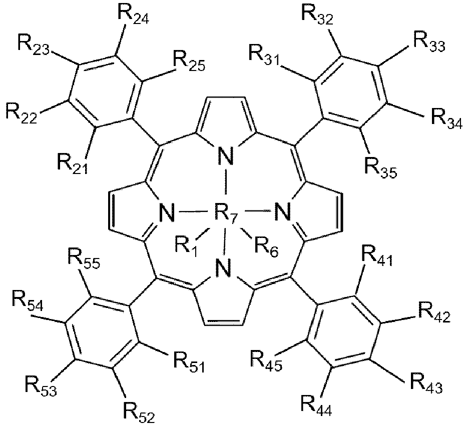 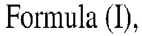 wherein R₆, which may be present or absent, is an optional group -Z, wherein group -Z is as follows: 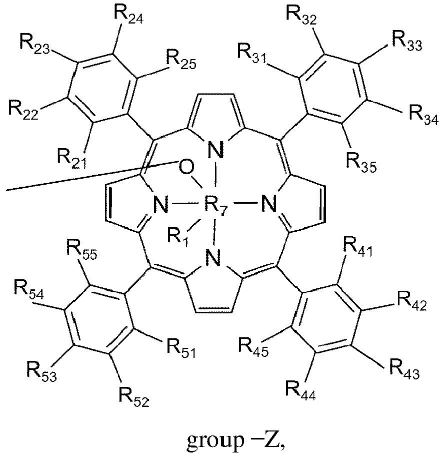 wherein, for formula (I) and for group -Z:
- R₁, which may be present or absent, is an optional anion selected from the group consisting of: Cl⁻, Br⁻, HO⁻ and CH₃COO⁻ ;
- R₂₁, R₂₂, R₂₃, R₂₄, R₂₅, R₃₁, R₃₂, R₃₃, R₃₄, R₃₅, R₄₁, R₄₂, R₄₃, R₄₄, R₄₅, R₅₁, R₅₂, R₅₃, R₅₄ and R₅₅ are selected, independently from each other, from the group consisting of: -H, -F, -CO₂H, -SO₃H and -H₂PO₃; and
- R₇ is selected from the group consisting of: -Fe and -Ni,

wherein, in formula (I),
when R₇ is -Ni, R₆ is always absent and R₁ is present if R₇ is -Ni(III) and R₁ is absent if R₇ is -Ni(II).

Advantageously, R₇ is -Ni(II) and R₁ and R₆ are absent.

Advantageously, R₇ is -Fe and R₆ is absent.

Advantageously, formula (I) is the following formula (Ia1): 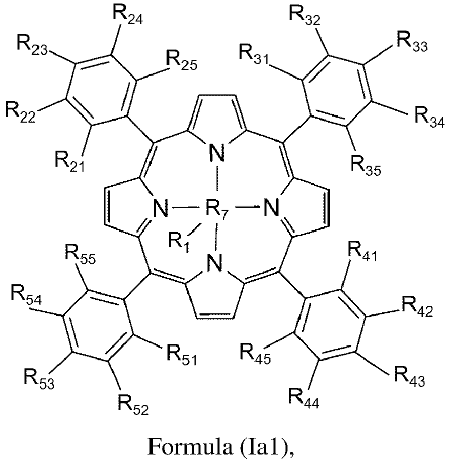 wherein R₁ is an anion selected from the group consisting of: Cl⁻, Br⁻, HO⁻ and CH₃COO⁻, preferably R₁ is Cl⁻;
wherein R₇ is selected from the group consisting of: -Fe and -Ni, and
wherein R₂₁, R₂₂, R₂₃, R₂₄, R₂₅, R₃₁, R₃₂, R₃₃, R₃₄, R₃₅, R₄₁, R₄₂, R₄₃, R₄₄, R₄₅, R₅₁, R₅₂, R₅₃, R₅₄ and R₅₅ are selected, independently from each other, from the group consisting of: -H, -F, -CO₂H, -SO₃H and -H₂PO₃.

Advantageously, formula (I) is the following formula (Ia2): 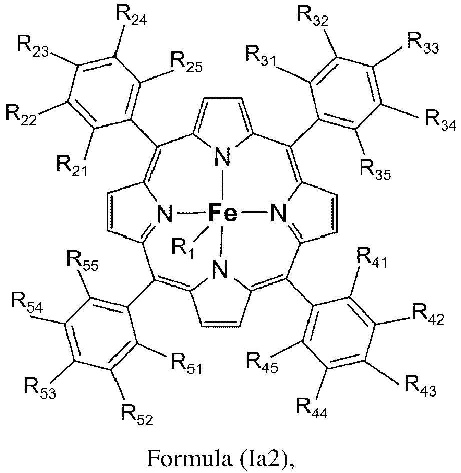 wherein R₁ is an anion selected from the group consisting of: Cl⁻, Br⁻, HO⁻ and CH₃COO⁻, preferably R₁ is Cl⁻; and
wherein R₂₁, R₂₂, R₂₃, R₂₄, R₂₅, R₃₁, R₃₂, R₃₃, R₃₄, R₃₅, R₄₁, R₄₂, R₄₃, R₄₄, R₄₅, R₅₁, R₅₂, R₅₃, R₅₄ and R₅₅ are selected, independently from each other, from the group consisting of: -H, -F, -CO₂H, -SO₃H and -H₂PO₃.

Advantageously, R₁ is Cl⁻.

Advantageously, R₃₁, R₃₂, R₃₃, R₃₄, R₃₅, R₄₁, R₄₂, R₄₃, R₄₄, R₄₅, R₅₁, R₅₂, R₅₃, R₅₄ and R₅₅ are each -F.

Advantageously, one moiety of the group (R₂₁, R₂₂, R₂₃, R₂₄, R₂₅) is -CO₂H and the four other moieties in this group are -H.

Advantageously, one moiety of the group (R₃₁, R₃₂, R₃₃, R₃₄, R₃₅) is -CO₂H and the four other moieties in this group are -H.

Advantageously, one moiety of the group (R₄₁, R₄₂, R₄₃, R₄₄, R₄₅) is -CO₂H and the four other moieties in this group are -H.

Advantageously, one moiety of the group (R₅₁, R₅₂, R₅₃, R₅₄, R₅₅) is -CO₂H and the four other moieties in this group are -H.

Advantageously, one moiety of each groups (R₂₁, R₂₂, R₂₃, R₂₄, R₂₅), (R₃₁, R₃₂, R₃₃, R₃₄, R₃₅), (R₄₁, R₄₂, R₄₃, R₄₄, R₄₅) and (R₅₁, R₅₂, R₅₃, R₅₄, R₅₅) is -CO₂H and the four other moieties in each group are -H.

Advantageously, one moiety of each groups (R₂₁, R₂₂, R₂₃, R₂₄, R₂₅), (R₃₁, R₃₂, R₃₃, R₃₄, R₃₅), (R₄₁, R₄₂, R₄₃, R₄₄, R₄₅) and (R₅₁, R₅₂, R₅₃, R₅₄, R₅₅) is -SO₃H and the four other moieties in each group are -H.

Advantageously, one moiety of each groups (R₂₁, R₂₂, R₂₃, R₂₄, R₂₅), (R₃₁, R₃₂, R₃₃, R₃₄, R₃₅), (R₄₁, R₄₂, R₄₃, R₄₄, R₄₅) and (R₅₁, R₅₂, R₅₃, R₅₄, R₅₅) is -H₂PO₃ and the four other moieties in each group are -H.

Advantageously, formula (I) is the following formula (Ia3): 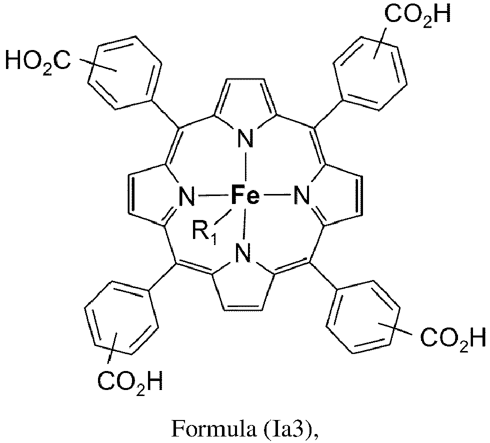 wherein R₁ is an anion selected from the group consisting of: Cl⁻, Br⁻, HO⁻ and CH₃COO⁻, preferably R₁ is Cl⁻.

In one embodiment, formula (I) is the following formula (Ib): 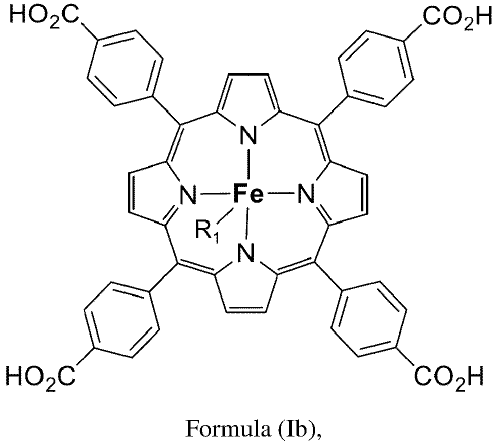 wherein R₁ is an anion selected from the group consisting of: Cl⁻, Br⁻, HO⁻ and CH₃COO⁻, preferably R₁ is Cl⁻.

In one embodiment, formula (I) is the following formula (Ic): 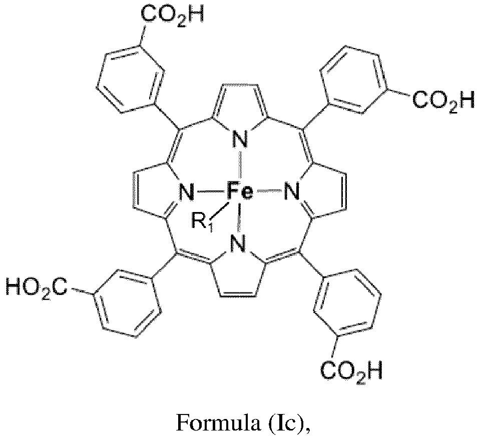 wherein R₁ is an anion selected from the group consisting of: Cl⁻, Br⁻, HO⁻ and CH₃COO⁻, preferably R₁ is Cl⁻.

In one embodiment, formula (I) is the following formula (Id): 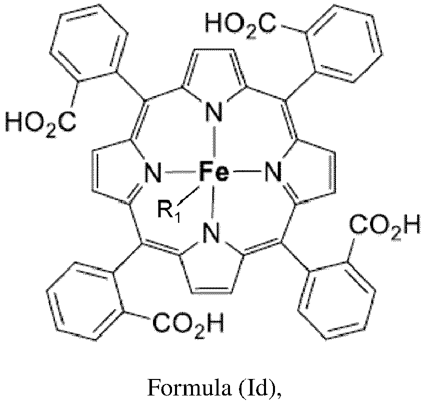 wherein R₁ is an anion selected from the group consisting of: Cl⁻, Br⁻, HO⁻ and CH₃COO⁻, preferably R₁ is Cl⁻.

Advantageously, formula (I) is selected from the group consisting of formula (Ib), formula (Ic), and formula (Id).

Advantageously, when R₇ is -Fe, the iron atom is an iron(III) atom, i.e. an iron atom in the +III oxidation state.

Advantageously, when R₇ is -Ni, the nickel atom is either a nickel(III) atom (also named -Ni(III)), i.e. a nickel atom in the +III oxidation state, or a nickel(II) atom, i.e. a nickel atom in the +II oxidation state (also named -Ni(II)). More advantageously, R₇ is - Ni(III) and R₁ is present. Alternatively, R₇ is -Ni(II) and R₁ is absent.

In one embodiment, formula (I) is the following formula (Ie):

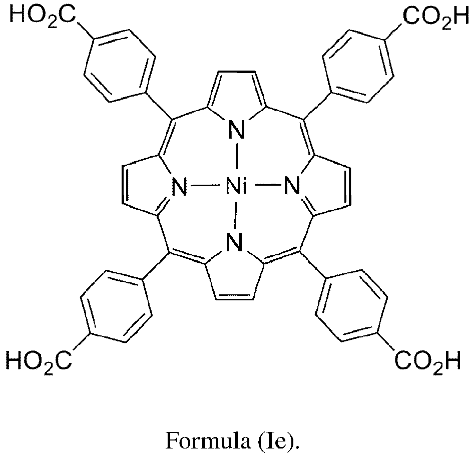

In one embodiment, formula (I) is the following formula (If):

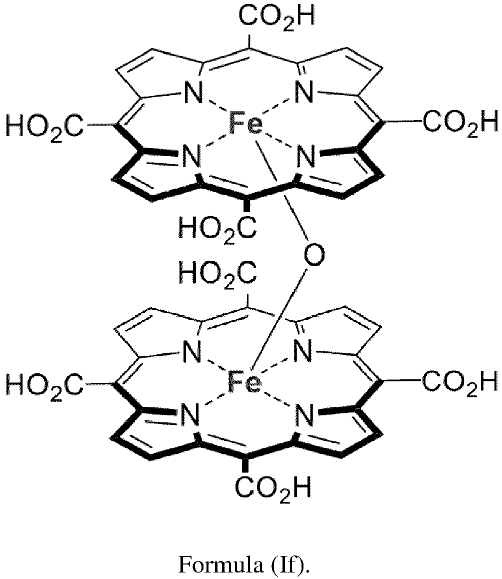

The alkene to be oxidized is either an aliphatic alkene or an aromatic alkene.

Advantageously, the alkene to be oxidized is an aliphatic alkene. More advantageously, the alkene to be oxidized is selected from the group consisting of: isoprene, 1-octene, trans-2-octene, cis-2-octene, 1-decene, 1-dodecene, cyclohexene, norbornene, methyl oleate, allylbenzene, 1-allyl-2-methylbenzene, allylcyclohexane, 1-allyl-4-(trifluoromethyl)benzene, propylene and mixtures thereof. Even more advantageously, the alkene to be oxidized is selected from the group consisting of 1-octene and propylene.

Advantageously, the alkene to be oxidized is an aromatic alkene. More advantageously, the alkene to be oxidized is selected from the group consisting of: styrene, tert-butylstyrene, 3-methyl-1*H*-indole, 4-methoxystyrene, 4-chlorostyrene, 4-bromostyrene, 4-trifluoromethyl-styrene, 4-methoxycarbonylstyrene, 4-carboxystyrene, 2,4-dimethylstyrene, 4-vinylpyridine and mixtures thereof. Even more advantageously, the alkene to be oxidized is styrene.

Advantageously, the alkene to be oxidized is selected from the group consisting of: isoprene, 1-octene, trans-2-octene, cis-2-octene, 1-decene, 1-dodecene, cyclohexene, norbornene, methyl oleate, allylbenzene, 1-allyl-2-methylbenzene, allylcyclohexane, 1-allyl-4-(trifluoromethyl)benzene, propylene, styrene, tert-butylstyrene, 3-methyl-1*H-*indole, 4-methoxystyrene, 4-chlorostyrene, 4-bromostyrene, 4-trifluoromethyl-styrene, 4-methoxycarbonylstyrene, 4-carboxystyrene, 2,4-dimethylstyrene, 4-vinylpyridine and mixtures thereof. Even more advantageously, the alkene to be oxidized is selected from the group consisting of: 1-octene, propylene, styrene and mixtures thereof.

Advantageously, the ketone to be synthesized is selected from the group consisting of: aromatic ketone, heteroaromatic ketone and aliphatic ketone. More advantageously, the ketone to be synthesized is selected from the group consisting of: acetophenone and acetone. Even more advantageously, the ketone to be synthesized is acetophenone.

All the features described below for a process of oxidation of an alkene into a ketone (part "Process of oxidation of an alkene into a ketone") apply *mutatis mutandis* for the use of a compound of formula (I) as a catalyst for the oxidation of an alkene into a ketone.

### Process of oxidation of an alkene into a ketone

The invention also relates to a process of oxidation of an alkene into a ketone comprising a step (a) of reacting said alkene with an oxidant in the presence of a catalyst, wherein said catalyst is a compound of formula (I) as described above.

All the features described above for the use of a compound of formula (I) (part "Use of a compound of formula (I) as a catalyst for the oxidation of an alkene into a ketone") apply *mutatis mutandis* for the process of oxidation of an alkene into a ketone, in particular the features related to formula (I), to the alkene to be oxidized and to the ketone to be synthesized.

Advantageously, step (a) is carried out at a temperature ranging from 0°C to 100°C. Preferably, step (a) is carried out at a temperature ranging from 0°C to 50°C. More preferably, step (a) is carried out at a temperature ranging from 15°C to 30°C. Even more preferably, step (a) is carried out at a temperature ranging from 20°C to 25°C, better at a temperature of about 20°C.

Indeed, the use of a compound of formula (I) as a catalyst during step (a) allows the oxidation of an alkene into a ketone at a temperature ranging from 0°C to 100°C. The reaction can be carried out at room temperature in an efficient way, as well as at 0°C or 100°C.

Advantageously, step (a) is carried out at atmospheric pressure.

Advantageously, step (a) is carried out at air or O₂ atmosphere, preferably at air atmosphere.

Indeed, the use of a compound of formula (I) as a catalyst during step (a) allows the oxidation of an alkene into a ketone at atmospheric pressure and without the need for an increase in dioxygen (O₂) pressure.

Advantageously, step (a) is carried out in the presence of at least one compound selected from the group consisting of: 1,1,3,3-tetramethyldisiloxane and compounds of the following formula (II):

R_{4-y}SiH_{y} (II),

wherein y is an integer ranging from 1 to 3 and R is selected from the group consisting of a C₁-C₆ alkyl and an aryl, preferably R is either an ethyl or a phenyl.

Advantageously, step (a) is carried out in the presence of at least one compound of the following formula (II):

R_{4-y}SiH_{y} (II),

wherein y is an integer ranging from 1 to 3 and R is selected from the group consisting of a C₁-C₆ alkyl and an aryl, preferably R is either an ethyl or a phenyl. More advantageously, step (a) is carried out in the presence of at least one compound selected from the group consisting of Et₃SiH, Ph₃SiH, Ph₂SiH₂ and PhSiH₃.

Advantageously, formula (I) is selected in the group consisting of formula (Ia2), formula (Ia3), formula (Ib), formula (Ic) and formula (Id) and step (a) is carried out in the presence of at least one compound of the following formula (II). More advantageously, formula (I) is formula (Ib) and step (a) is carried out in the presence of at least one compound of the following formula (II). Even more advantageously, formula (I) is formula (Ib) and step (a) is carried out in the presence of at least one compound selected in the group consisting of Et₃SiH and PhSiH₃. Better, formula (I) is formula (Ib) and step (a) is carried out in the presence of PhSiH₃.

For example, step (a) may be carried out in the presence of one, two, three or four types of compounds of formula (II). Preferably, step (a) may be carried out in the presence of one or two types of compounds of formula (II). More preferably, step (a) may be carried out in the presence of one type of compound of formula (II), for example PhSiH₃.

Advantageously, said compound of formula (II) may be selected from the group consisting of Et₃SiH, Ph₃SiH, Ph₂SiH₂ and PhSiH₃. More advantageously, said compound of formula (II) may be selected from the group consisting of Et₃SiH and PhSiH₃. Even more advantageously, said compound of formula (II) is Et₃SiH. Alternatively, said compound of formula (II) is PhSiH₃. Alternatively, step (a) is carried out in the presence of Et₃SiH and PhSiH₃.

Advantageously, said compound of formula (II) is present in an amount (in mole) ranging from 2 to 3 equivalents with regards to the amount (in mole) of the alkene. More advantageously, said compound of formula (II) is present in an amount (in mole) of 3 equivalents with regards to the amount (in mole) of the alkene.

Advantageously, step (a) is carried out in the presence of 1,1,3,3-tetramethyldisiloxane. Advantageously, the 1,1,3,3-tetramethyldisiloxane is present in an amount (in mole) ranging from 2 to 3 equivalents with regards to the amount (in mole) of the alkene. More advantageously, the 1,1,3,3-tetramethyldisiloxane is present in an amount (in mole) of 3 equivalents with regards to the amount (in mole) of the alkene.

Advantageously, step (a) is carried out in the presence of a polymethylhydrosiloxane, preferably a polymethylhydrosiloxane having a weight average molar mass ranging from 5,000 g.mol⁻¹ to 400,000 g.mol⁻¹.

Advantageously, formula (I) is selected in the group consisting of formula (Ia1) wherein R₇ is - Ni and formula (Ie) and step (a) is carried out in the presence of polymethylhydrosiloxane, preferably a polymethylhydrosiloxane having a weight average molar mass ranging from 5,000 g.mol⁻¹ to 400,000 g.mol⁻¹. More advantageously, formula (I) is formula (Ie) and step (a) is carried out in the presence of polymethylhydrosiloxane, preferably a polymethylhydrosiloxane having a weight average molar mass ranging from 5,000 g.mol⁻¹ to 400,000 g.mol⁻¹.

Advantageously, the oxidant is selected from the group consisting of O₂, H₂O₂, and potassium peroxymonosulfate (oxone). More advantageously, the oxidant is O₂.

Advantageously, the molar concentration of the compound of formula (I) may range from 0.0001 % to 10 %, preferably from 1% to 7.5 %, more preferably about 2.5 %, the percentages being expressed relative to the molar concentration of the alkene to be oxidized. Otherwise expressed, the catalyst loading of the compound of formula (I) may range from 0.0001 mol % to 10 mol %, preferably from 1 mol % to 7.5 mol %, more preferably about 2.5 mol %.

The alkene and the compound of formula (I) may be either in the same phase or in different phases during step (a).

Advantageously, the alkene and the compound of formula (I) are in the same phase during step (a). More advantageously, step (a) is carried out in a homogeneous liquid medium comprising a solvent comprising at least 1% v/v, preferably from 1% v/v to 10% v/v, more preferably from 2% v/v to 10% v/v, even more preferably about 4% v/v, of water. Even more advantageously, step (a) is carried out in a homogeneous liquid medium comprising an alcohol-based solvent comprising at least 1% v/v, preferably from 1% v/v to 10% v/v, more preferably from 2% v/v to 10% v/v, even more preferably about 4% v/v, of water. Better, step (a) is carried out in a homogeneous liquid medium comprising ethanol as solvent, said ethanol comprising at least 1% v/v, preferably from 1% v/v to 10% v/v, more preferably from 2% v/v to 10% v/v, even more preferably about 4% v/v, of water.

Advantageously, the alkene and the compound of formula (I) are in the same phase during step (a). More advantageously, step (a) is carried out in an alcohol-based solvent, even more advantageously in ethanol, better in ethanol comprising at least 1% v/v, preferably from 1% v/v to 10% v/v, more preferably from 2% v/v to 10% v/v, even more preferably about 4% v/v, of water.

Advantageously, the alkene and the compound of formula (I) are in the same phase during step (a) and the compound of formula (I) is selected from the group consisting of the compound of formula (Ib), the compound of formula (Ic), the compound of formula (Id), the compound of formula (Ie) and the compound of formula (If), preferably the compound of formula (I) is the compound of formula (Ib) wherein R₁ is Cl⁻. More advantageously:
- the compound of formula (I) is selected from the group consisting of the compound of formula (Ib), the compound of formula (Ic), the compound of formula (Id), the compound of formula (Ie) and the compound of formula (If), preferably the compound of formula (I) is the compound of formula (Ib) wherein R₁ is Cl⁻; and
- the alkene and the compound of formula (I) are in the same phase during step (a), wherein step (a) is carried out in an alcohol-based solvent, even more advantageously in ethanol, better in ethanol comprising at least 1% v/v, preferably from 1% v/v to 10% v/v, more preferably from 2% v/v to 10% v/v, even more preferably about 4% v/v, of water.

Advantageously, the alkene and the compound of formula (I) are in the same phase during step (a) and the compound of formula (I) is selected from the group consisting of the compound of formula (Ib), the compound of formula (Ic) and the compound of formula (Id), preferably the compound of formula (I) is the compound of formula (Ib) wherein R₁ is Cl⁻. More advantageously:
- the compound of formula (I) is selected from the group consisting of the compound of formula (Ib), the compound of formula (Ic) and the compound of formula (Id), preferably the compound of formula (I) is the compound of formula (Ib) wherein R₁ is Cl⁻; and
- the alkene and the compound of formula (I) are in the same phase during step (a), wherein step (a) is carried out in an alcohol-based solvent, even more advantageously in ethanol, better in ethanol comprising at least 1% v/v, preferably from 1% v/v to 10% v/v, more preferably from 2% v/v to 10% v/v, even more preferably about 4% v/v, of water.

Advantageously, the alkene and the compound of formula (I) are in the same phase during step (a) and the compound of formula (I) is the compound of formula (Ie). More advantageously:
- the compound of formula (I) is the compound of formula (Ie); and
- the alkene and the compound of formula (I) are in the same phase during step (a), wherein step (a) is carried out in an alcohol-based solvent, even more advantageously in ethanol, better in ethanol comprising at least 1% v/v, preferably from 1% v/v to 10% v/v, more preferably from 2% v/v to 10% v/v, even more preferably about 4% v/v, of water.

Advantageously, the alkene and the compound of formula (I) are in the same phase during step (a) and the compound of formula (I) is the compound of formula (If). More advantageously:
- the compound of formula (I) is the compound of formula (If); and
- the alkene and the compound of formula (I) are in the same phase during step (a), wherein step (a) is carried out in an alcohol-based solvent, even more advantageously in ethanol, better in ethanol comprising at least 1% v/v, preferably from 1% v/v to 10% v/v, more preferably from 2% v/v to 10% v/v, even more preferably about 4% v/v, of water.

Alternatively, the alkene and the compound of formula (I) may be in different phases during step (a). More advantageously, the compound of formula (I) is integrated in a system selected from the group consisting of: metal organic framework (MOF), covalent organic framework (COF), metal organic cages (MOC), resins, surfaces, matrixes, waxes, silica, and alumina.

For example, step (a) may be an heterogenous catalysis and the compound of formula (I) integrated in a system selected from the group consisting of: metal organic framework (MOF) and covalent organic framework (COF).

Advantageously, step (a) may be carried out in batch or in flow.

In one embodiment, step (a) is carried out at room temperature (in particular at 20°C), in an homogenous medium comprising ethanol (preferably ethanol comprising about 4% v/v of water), in the presence of: air atmosphere, a compound of formula (I) (for example about 2.5 moles of a compound of formula (I) per cent moles of alkene), and from 2 to 3 molar equivalents of a compound of formula (II) relative to the amount of alkene.

Advantageously, step (a) may be as follows: 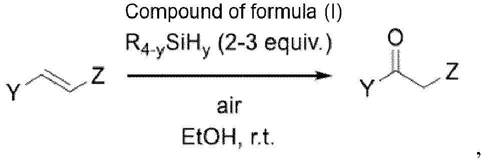 wherein formula (I) is advantageously selected from the group consisting of formula (Ib), formula (Ic), and formula (Id),
wherein the air atmosphere may be replaced by O₂ atmosphere,
wherein in the formula R_{4-y}SiH_{y}, R may advantageously be an ethyl or a phenyl, and
wherein Y and Z may advantageously be, independently from each other, selected from the group consisting of an alkyl, an aryl or any other functional moiety.

Advantageously, the process of oxidation of an alkene into a ketone comprises:
- a step (a), as described above, of reacting said alkene with an oxidant in the presence of a catalyst, wherein said catalyst is a compound of formula (I) as described above, and then
- a step (b) of recovering the compound of formula (I).

Advantageously, during step (b), the compound of formula (I) is recovered by precipitation. Preferably, during step (b), the compound of formula (I) is recovered by acid/base precipitation. Therefore, the compound of formula (I) may be recovered and recycled at the end of the reaction of oxidation of an alkene into a ketone, for example by precipitation.

Advantageously, during step (b), the compound of formula (I) is recovered by precipitation followed by a filtration. Preferably, during step (b), the compound of formula (I) is recovered by acid/base precipitation followed by a filtration. Therefore, the compound of formula (I) may be recovered and recycled at the end of the reaction of oxidation of an alkene into a ketone, for example by precipitation followed by a filtration.

Alternatively, during step (b), the compound of formula (I) may be recovered by extraction, for example from immiscible solvents of different polarities.

Thus, during step (b), the compound of formula (I) may be recovered by filtration, precipitation and/or extraction. The precipitation may for example be an acid/base precipitation. The extraction may for example be carried out using immiscible solvents of different polarities.

The process according to the invention allows the oxidation of alkenes into ketones under "mild" conditions, in particular it does not require oxygen pressure, a high temperature and/or the presence of carcinogenic compounds such as benzene, ethylbenzene or cumene. In addition, the compound of formula (I) has a high catalytic activity which allows a lower amount of said compound of formula (I) to be used. Furthermore, the compound of formula (I) can be used in both homogeneous and heterogeneous catalysis and allows a high yield of ketones to be synthesized with an increased selectivity with respect to the secondary products (alcohols).

### EXAMPLES

The present invention is further illustrated by the following examples.

In all the examples, solvents were purified with an MB SPS-800 purification system. Pyrrole was dried with CaH₂ and distilled prior to use. CDCl₃ was filtered through alumina and stored under argon over molecular sieves. All the other employed chemicals were purchased from commercial sources and used as received. Unless otherwise specified, reactions were carried out under argon atmosphere by employing standard Schlenk and vacuum-line techniques. ¹H and ¹³C NMR spectra were recorded with a Bruker GPX (400 MHz) spectrometer. ¹H NMR spectra were referenced to residual protiated solvent (δ = 7.26 ppm for CDCl₃). ¹³C NMR spectra were referenced to CDCl₃ (δ = 77.16 ppm). Abbreviations for signal couplings are: br, broad; s, singlet; d, doublet; t, triplet; m, multiplet; dd, doublet of doublets; dt, triplet of doublets; td, doublet of triplets; tt, triplet of triplets; tdd, doublet of doublet of triplets. Coupling constants, J, were reported in hertz unit (Hz). The reactions were monitored by using a Shimadzu 2014 gas chromatograph equipped with an EquityTM-1 Fused Silica capillary column (30 m x 0.25 mm x 0.25 µm) and an FID detector; conversion was determined by using dodecane as internal standard. UV/Vis absorption spectra were recorded with a Specord 205 UV/Vis/NIR spectrophotometer and quartz cuvettes of 1 cm path length.

### Example 1: Synthesis of a compound of formula (Id) according to the invention

### Materials and Methods

A compound according to formula (Id) according to the invention was synthetized: the compound named "Fe-C1". The reaction mechanism that was used is the following:

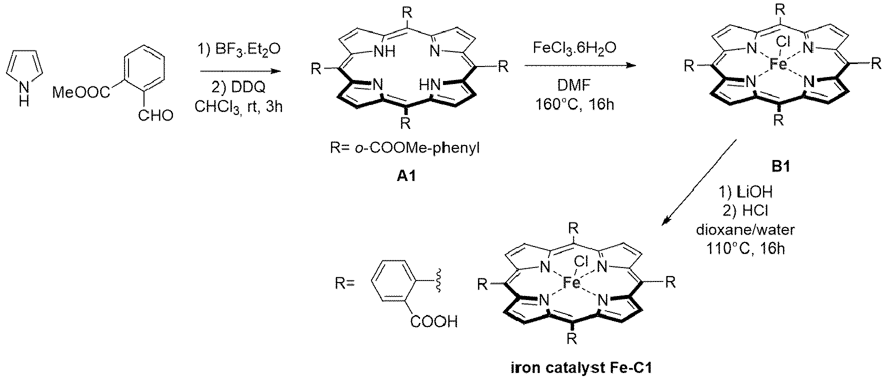

### Results

**Synthesis and characterization of A1:** Distilled pyrrole (0.34 g, 0.35 mL, 5.0 mmol 1 equiv.) and methyl 2-formylbenzoate (0.83 mg, 0.7 mL, 5.0 mmol, 1 equiv.) were added to a pre-dried round bottom flask under argon atmosphere. DCM (500 mL) was added, the reaction mixture was covered with aluminum foil and BF3. Et₂O (0.31 mL, 2.5 mmol, 0.5 equiv.) was added. The reaction mixture was stirred for 2.5h at room temperature protected from light, then DDQ (1.13 g, 5.0 mmol, 1 equiv.) was added and the reaction mixture was stirred for another 16 h. Then Et₃N was added to quench the reaction and after 5 min, the solvent was evaporated under vacuum. The crude mixture was purified by SiO₂ column chromatography (heptane:DCM 9:1 to 1:9) affording **A1** as a purple powder. The multiple fraction containing the four atropoisomers were collected and evaporated yielding the product as purple crystals (0.4 g, 38% yield). ¹H NMR (400 MHz, CDCl₃): δ = 8.65-7.59 (m, 24H), 2.89 (s, 3H), 2.78 (s, 3H), 2.73 (s, 3H), 2.61 (s, 3H), -2.42 (s, 2H) ppm. The data match those found in the literature.

**Synthesis and characterization of B1:** A1 (0.16 g, 0.19 mmol, 1 equiv.), FeCl₂·4H₂O (0.47 g, 2.4 mmol, 13 equiv.) and DMF (20 mL) were introduced in a round bottom flask equipped with a stirring bar. The reaction mixture was refluxed for 16 h at 160 °C, then the solvent was slowly evaporated at 80°C over 1 hour until all residual DMF was removed from the reaction mixture. Back at room temperature, the crude was washed with HCl (1M) and water until the aqueous phase became colorless, then the solid was dissolved in dichloromethane. The organic layer was dried with MgSO4, filtered and the solvent was evaporated under vacuum yielding the pure product (0.18 g, 99% yield). ¹H NMR (400 MHz, CDCl₃): δ = 80.4, 14.4, 13.7, 13.5, 12.4. λabs/nm (DMF), (ε/103 M⁻¹ cm⁻¹): 421 (78.1), 519 (7.25), 577 (6.62). The data match those found in the literature.

**Synthesis and characterization of Fe-C1:** B1 (1.450 g, 1.55 mmol, 1 equiv.), LiOH (3,72 g, 155 mmol, 100 equiv.) and dioxane/water (7/1): 150 mL were introduced in a round bottom flask equipped with a stirring bar. The reaction mixture was refluxed for 16 h at 110 °C, then the solvent was evaporated and the product was precipitated by HCl (1M) at pH = 1 (followed by pH paper). The solution was filtrated and the solid was washed with HCl (1M), water, heptane and dichloromethane and then dried under vacuum yielding the pure product (1.38 g, 99% yield). ¹H NMR (400 MHz, DMSO-d₆): δ = 73.4, 10.1, 9.7, 9.3. λabs /nm (DMF), (ε/103 M⁻¹ cm⁻¹): 426 (17.5), 515 (2.64), 573 (1.42). HRMS (ESI) calcd. for [M-Cl] + C48H28N4O8⁵⁶Fe 844.1251, found 844.1249 (0 ppm).

### Example 2: Synthesis of a compound of formula (Ic) according to the invention

### Materials and Methods

A compound according to formula (Ic) according to the invention was synthetized: compound named "Fe-C2". The reaction mechanism that was used is the following:

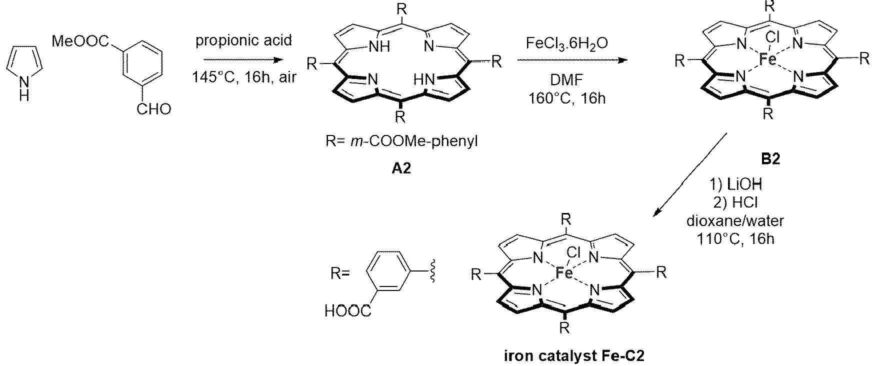

### Results

**Synthesis and characterization of A2:** Distilled pyrrole (2 g, 2.07 mL, 30 mmol, 1 equiv.) and methyl 3-formylbenzoate (5 g, 30 mmol, 1 equiv.) were added to a pre-dried round bottom flask containing propionic acid (150 mL). The reaction mixture was covered with aluminum foil and the reaction mixture was refluxed for 16 h at 145 °C. After cooling to room temperature, the resulting purple crystals were filtered and washed with water and then dissolved in dichloromethane. The organic layer was dried with MgSO₄, filtered and the solvent was evaporated affording a crude mixture that was purified by SiO₂ column chromatography (heptane:DCM 9:1 to 1:9). The filtrate was then evaporated under vacuum yielding the pure product (1.21 g, 19% yield). ¹H NMR (400 MHz, CDCl₃): δ = 8.92 (s, 4H), 8.82 (s, 8H), 8.52 (d, J = 8 Hz, 4H), 8.43 (d, J = 8 Hz, 4H), 7.88 (t, J = 7.6 Hz, 4H), 4.02 (s, 12H), -2.77 (s, 2H) ppm. The data match those found in the literature.

**Synthesis and characterization of B2:** A2 (0.43 g, 0.5 mmol, 1 equiv.), FeCl₂·6H₂O (1.24 g, 6.5 mmol, 13 equiv.) and DMF (30 mL) were introduced in a round bottom flask equipped with a stirring bar. The reaction mixture was refluxed for 16 h at 160 °C, then the solvent was slowly evaporated at 80°C over 1 hour until all residual DMF was removed from the reaction mixture. Back at room temperature, the crude was washed with HCl (1M) and water until the aqueous phase became colorless, then the solid was dissolved in dichloromethane. The organic layer was dried with MgSO4, filtered and the solvent was evaporated under vacuum yielding the pure product (0.42 g, 99% yield). ¹H NMR (400 MHz, CDCl₃): δ = 80.3, 13.3, 12.2, 8.39, 7.72 ppm. λabs /nm (DMF), (ε/103 M⁻¹ cm⁻¹): 424 (92.6), 520 (7.59), 569 (11.2). The data match those found in the literature.

**Synthesis and characterization of Fe-C2:** B2 (0.26 g, 0.28 mmol, 1 equiv.), LiOH (1,32 g, 55 mmol, 200 equiv.) and dioxane/water (7/1): 68 mL were introduced in a round bottom flask equipped with a stirring bar. The reaction mixture was refluxed for 16 h at 105 °C, then the solvent was evaporated and the product was precipitated by HCl (1M) at pH = 1 (followed by pH paper). The solution was filtrated and the solid was washed with HCl (1M), water, heptane and dichloromethane and then dried under vacuum yielding the pure product (0.24 g, 99% yield). ¹H NMR (400 MHz, DMSO-d₆): δ = 80.3, 13.6, 13.3, 12.2 ppm. HRMS-ESI calcd for C48H28FeN4O8 [M-Cl] + : 844.12565, found 844.1249 (0 ppm). λabs /nm (DMF), (ε/103 M-1 cm-1): 416 (62.4), 522 (5.51), 573 (5.26). HRMS (ESI) calcd. for [M-Cl] + C₄₈H₂₈N₄O₈⁵⁶Fe 844.1251, found 844.1249 (0 ppm).

### Example 3: Synthesis of a compound of formula (Ib) according to the invention

### Materials and Methods

A compound according to formula (Ib) according to the invention was synthetized: compound named "Fe-C3". The reaction mechanism that was used is the following:

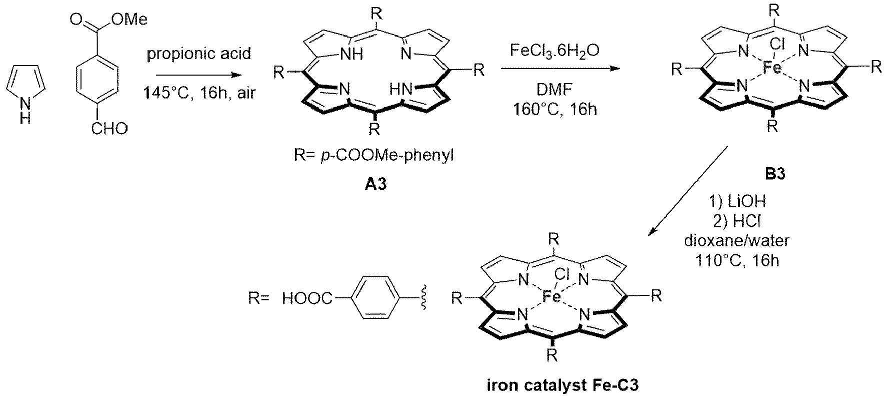

### Results

**Synthesis and characterization of A3:** Distilled pyrrole (1 g, 1.04 mL, 15 mmol, 1 equiv.) and methyl 4-formylbenzoate (2.48 g, 15 mmol, 1 equiv.) were added to a pre-dried round bottom flask containing propionic acid (75 mL). The reaction mixture was covered with aluminum foil and the reaction mixture was refluxed for 16 h at 145 °C. After cooling to room temperature, the resulting purple crystals were filtered and washed with water and then dissolved in dichloromethane. The organic layer was dried with MgSO4, filtered and the solvent was evaporated affording a crude mixture that was purified by filtration on neutral alumina with dichloromethane as the eluant. The filtrate was then evaporated under vacuum yielding the pure product (1.14 g, 34% yield). ¹H NMR (400 MHz, CDCl₃): δ = 8.87 (s, 8H), 8.49 (d, J = 8.1, 8H), 8.33 (d, J = 8.1, 8H), 4.15 (s, 12H), -2.74 (s, 2H) ppm. The data match those found in the literature.

**Synthesis and characterization of B3:** A3 (1.14 g, 5.1 mmol, 1 equiv.), FeCl₂·4H₂O (13.1 g, 66 mmol, 13 equiv.) and DMF (150 mL) were introduced in a round bottom flask equipped with a stirring bar. The reaction mixture was refluxed for 16 h at 160 °C, then the solvent was slowly evaporated at 80°C over 1 hour until all residual DMF was removed from the reaction mixture. Back at room temperature, the crude was washed with HCl (1M) and water until the aqueous phase became colorless, then the solid was dissolved in dichloromethane. The organic layer was dried with MgSO4, filtered and the solvent was evaporated under vacuum yielding the pure product (0.870, g, 87% yield). ¹H NMR (400 MHz, CDCl₃): δ = 80.4, 14.0, 12.9 ppm. λabs /nm (DMF), (ε/10 3 M⁻¹ cm⁻¹): 428 (109), 520 (7.25), 571 (8.47). The data match those found in the literature.

**Synthesis and characterization of Fe-C3:** B3 (0.870 g, 4.44 mmol, 1 equiv.), LiOH (32 g, 1.33 mol, 300 equiv.) and dioxane/water (7/1): 250 mL were introduced in a round bottom flask equipped with a stirring bar. The reaction mixture was refluxed for 16 h at 110 °C, then the solvent was evaporated and the product was precipitated by HCl (1M) at pH = 1 (followed by pH paper). The solution was filtrated and the solid was washed with HCl (1M), water, heptane and dichloromethane and then dried under vacuum yielding the pure product (0.745 g, 90% yield). ¹H NMR (400 MHz, DMSO-d₆): δ = 80, 74.1, 13.8, 10.0 ppm. λ abs /nm (DMF), (ε/103 M⁻¹.cm⁻¹): 415 (28.8), 522 (6.52), 571 (4.55). HRMS (ESI) calcd. for [M-Cl]+ C₄₈H₂₈N₄O₈⁵⁶Fe 844.1251, found 844.1249 (0 ppm).

### Example 4: Synthesis of a compound of formula (Ie) according to the invention

### Materials and Methods

A compound according to formula (Ie) according to the invention was synthetized: the compound named "Ni-1". The reaction mechanism that was used is the following:

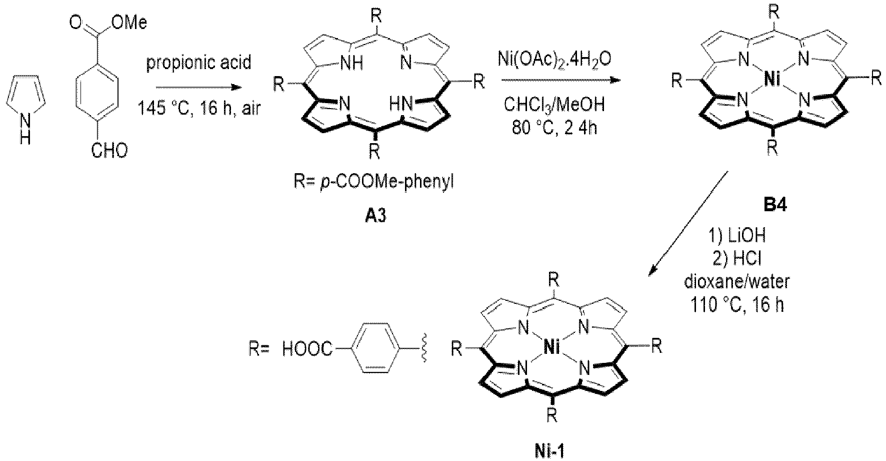

**Synthesis and characterization of A3:** Distilled pyrrole (2 g, 2.07 mL, 30 mmol, 1 equiv.) and methyl 3-formylbenzoate (5 g, 30 mmol, 1 equiv.) were added to a pre-dried round bottom flask containing propionic acid (150 mL). The reaction mixture was covered with aluminum foil and the reaction mixture was refluxed for 16 h at 145 °C. After cooling to room temperature, the resulting purple crystals were filtered and washed with water and then dissolved in dichloromethane. The organic layer was dried with MgSO₄, filtered and the solvent was evaporated affording a crude mixture that was purified by SiO₂ column chromatography (heptane:DCM 9:1 to 1:9). The filtrate was then evaporated under vacuum yielding the pure product (1.21 g, 19% yield). ¹H NMR (400 MHz, CDCl₃): δ = 8.92 (s, 4H), 8.82 (s, 8H), 8.52 (d, J = 8 Hz, 4H), 8.43 (d, J = 8 Hz, 4H), 7.88 (t, J = 7.6 Hz, 4H), 4.02 (s, 12H), -2.77 (s, 2H) ppm.

**Synthesis and characterization of B4:** A3 (0.052 g, 0.06 mmol, 1 equiv.), Ni(OAc)₂·4H₂O (0.200 g, 0.8 mmol, 13 equiv.) and CHCl₃/MeOH (20 mL, 4:1) were introduced in a round bottom flask equipped with a stirring bar and a condenser. The reaction mixture was refluxed for 24 h at 80 °C. Then the solvent was evaporated and the crude was washed with HCl (1M) and water until the aqueous phase became colorless, then the solid was dissolved back in dichloromethane. The organic layer was dried with MgSO4, filtered and the solvent was evaporated under vacuum yielding the pure product (0.053 g, 95% yield). ¹H NMR (400 MHz, Chloroform-d) δ = 8.70 (s, 8H), 8.37 (s, 8H), 8.09 (s, 8H), 4.08 (s, 12H).

**Synthesis and characterization of Ni-1:** B4 (1.000 g, 1.11 mmol, 1 equiv.), LiOH (2.64 g, 0.111 mol, 100 equiv.) and dioxane/water (7/1): 250 mL were introduced in a round bottom flask equipped with a stirring bar. The reaction mixture was refluxed for 16 h at 110 °C, then the solvent was evaporated and the product was precipitated by HCl (1M) at pH = 1 (followed by pH paper). The solution was filtrated and the solid was washed with HCl (1M), water, heptane and dichloromethane and then dried under vacuum yielding the pure product (0.811 g, 87% yield). ¹H NMR (400 MHz, DMSO-*d*₆) δ = 8.74 (s, 8H), 8.21 (AB, J = 76.5, 16H). HRMS (ESI; CH₂Cl₂ / CH₃OH: 95/5) calcd. for [M-H]⁻ C₄₈H₂₇N₄O₈⁵⁸Ni 845.11879, found 845.1185 (0 ppm).

### Example 5: Synthesis of a compound of formula (If) according to the invention

### Materials and Methods

A compound according to formula (If) according to the invention was synthetized: the compound named "Fe-4". The reaction mechanism that was used is the following:

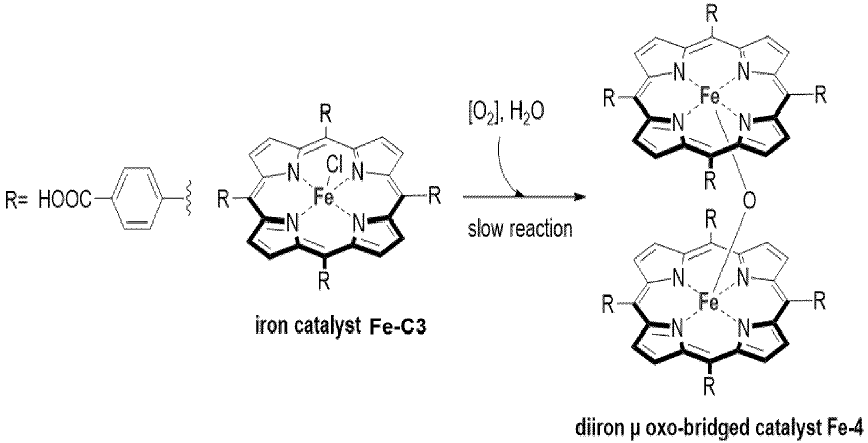

**Synthesis and characterization of Fe-4:** Fe-C3 (synthesized in Example 3) was stored under air for 3 months at room temperature and was added (14.2 mg, 0.016 mmol, 0.05 equiv.) to and oven dried Schlenk flask, EtOH/H₂O (96/4) (3mL), tert-butylstyrene (52 mg, 60 µL, 0.325 mmol, 1 equiv.), Et3SiH (76 mg, 104 µL, 0.650 mmol, 2 equiv.) and dodecane (0.081 mmol, 0.25 equiv.). A balloon filled with pure oxygen was added onto the Schlenk and the reaction mixture was stirred at room temperature for 24 hours. ¹H NMR (400 MHz, DMSO-*d*₆) δ = 79.63, 73.92, 13.66, 12.77, 11.23, 10.39, 9.97, 8.38, 7.67. HRMS (MALDI): m/z calcd for C₉₆H₅₆N₈O₁₇⁵⁶Fe₂ 1704.24567 [M]^{+.}; found: 1704.267 (12 ppm). Other species detected that can be originated from the fragmentation of the µ oxo-bridged iron catalyst as well as being in the catalyst batch mixture: m/z calcd for C₄₈H₂₈N₄O₈⁵⁶Fe 844.1251 [M]⁺; found: 844.126 (1 ppm); m/z calcd for C₄₈H₂₈N₄O₈³⁵Cl⁵⁶Fe 879.09396 [M]⁺; found: 879.095 (1 ppm).

### Example 6: Oxidation of alkene using compounds of formula (I) according to the invention as catalyst

### Materials and Methods

In five different oven dried Schlenk flasks were added either:
- compound Fe-C2 (synthesized in Example 2 of the present application),
- compound Fe-C3 (synthesized in Example 3 of the present application),
- compound A2 (synthesized in Example 2 of the present application),
- compound B2 (synthesized in Example 2 of the present application), and
- compound Fe-C1 (synthesized in Example 1 of the present application).

In each Schlenk flask were also added: (EtOH/H₂O) (96/4) (3mL), tert-butylstyrene (52 mg, 60 µL, 0.325 mmol, 1 equiv.), Et₃SiH (113 mg, 156 µL, 0.975 mmol, 3 equiv.) and dodecane (0.081 mmol, 0.25 equiv.). A round-bottom flask filled with pure oxygen was added onto each Schlenk flask and the reaction mixtures were stirred at room temperature for 18 h. For the five reactions, the mechanism was as follows:

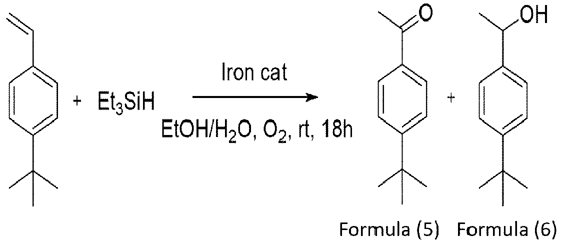

The iron catalyst ("Fe cat") was either compound Fe-C2, Fe-C3, A2, B2 or Fe-C1. "rt" = room temperature.

### Results

For the five reactions, the catalyst loading was of 7.5 mol %.

The results are presented in the following table:

**Table 1**

| Catalyst | Conversion of tert-butylstyrene (% w/w relative to the total weight of the tert-butylstyrene)* | Compound of formula (5) produced (% w/w relative to the total weight of the products)* | Compound of formula (6) produced (% w/w relative to the total weight of the products)* |
|---|---|---|---|
| A2 | 0 | 0 | 0 |
| B2 | 0 | 0 | 0 |
| Fe-C 1 | 29 | 27 | 2 |
| Fe-C2 | 93 | 90 | 3 |
| Fe-C3 | 100 | 98 | 2 1 |

| | | | |
|---|---|---|---|
| *Calculated by GC-MS | | | |

Thus, the above results show that the use of a compound of formula (I) according to the invention results in a good conversion of tert-butylstyrene to ketone as the main product. This conversion is even better when the Fe-C3 compound is used, with 100% w/w of the tert-butylstyrene converted into products, among which 98% w/w were the ketone of formula (5) relative to the total weight of the products.

Therefore, the use of the compounds of formula (I) according to the invention allows a highly selective oxidation of an alkene into a ketone.

### Example 7: Oxidation of alkene using compound of formula (Ie) according to the invention as catalyst

### Materials and Methods

To an oven dried Schlenk flask was added Ni-1 (synthesized in Example 4 of the present application), undistilled EtOH, tert-butylstyrene, silane and dodecane. A balloon filled with air was added onto the Schlenk and the reaction mixture was stirred at 20°C for 4.5 or 24 hours. Conversion and estimated yield were monitored by GC analysis using dodecane as the internal standard. The reaction mechanism was as follows:

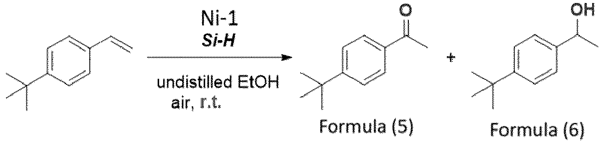

Si-H was either polymethylhydroxysilane (PMHS) or PhSiH₃. "rt" = room temperature.

### Results

The results are presented in the following table:

**Table 2**

| Silane (corresponding to Si-H) | Time (hours) | Conversion of tert-butylstyrene (% w/w relative to the total weight of the tert-butylstyrene)* | Compound of formula (5) produced (% w/w relative to the total weight of the products)* | Compound of formula (6) produced (% w/w relative to the total weight of the products)^{∗} |
|---|---|---|---|---|
| PMHS | 24 | 100 | 79 | 21 |
| PhSiH₃ | 4.5 | 100 | 86 | 14 |

| | | | | |
|---|---|---|---|---|
| *Calculated by GC-MS | | | | |

Therefore, the use of compound of formula (Ie) and thus of the compounds of formula (I) according to the invention allows a highly selective oxidation of an alkene into a ketone.

### Example 8: Oxidation of alkene using compound of formula (If) according to the invention as catalyst

### Materials and Methods

To an oven dried Schlenk flask was added Fe-4 (synthesized in Example 5 of the present application) (14.2 mg, 0.016 mmol, 0.025 equiv.), EtOH/H₂O (96/4) (3mL), tert-butylstyrene (52 mg, 60 µL, 0.325 mmol, 1 equiv.), silane, dodecane (0.081 mmol, 0.25 equiv.) and optionally an additive. A balloon filled with air was added onto the Schlenk and the reaction mixture was stirred at room temperature for a given time. Yield (%) was monitored by GC analysis using dodecane as the internal standard. The reaction mechanism was as follows:

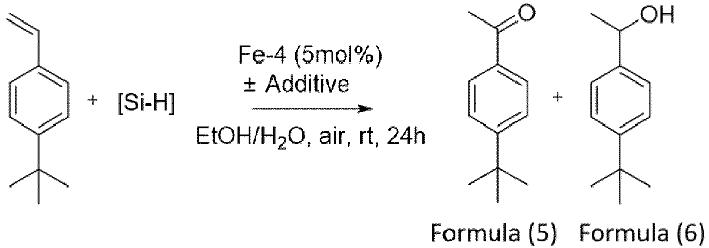

### Results

The results are presented in the following table:

**Table 3**

| Silane (corresponding to [Si-H]) | Additive | Time (hours) | Conversion of tert-butylstyrene (% w/w relative to the total weight of the tert-butylstyrene)* | Compound of formula (5) produced (% w/w relative to the total weight of the products)* | Compound of formula (6) produced (% w/w relative to the total weight of the products)* |
|---|---|---|---|---|---|
| PhSiH₃ | - | 5 | 100 | 92 | 8 |
| Ph₂SiH₂ | - | 5 | 100 | 91 | 9 |
| Et₃SiH (3 equiv.) | HCl (10 mol%) | 24 | 60 | 48 | 12 |
| Et₃SiH (3 equiv.) | HCl (20 mol%) | 24 | 62 | 49 | 13 |
| Et₃SiH (3 equiv.) | PhSiH₃ (20 mol%) | 24 | 100 | 98 | 2 |

| | | | | | |
|---|---|---|---|---|---|
| *Calculated by GC-MS | | | | | |

Therefore, the use of compound of formula (If) and thus of the compounds of formula (I) according to the invention allows a highly selective oxidation of an alkene into a ketone.

### Example 9: Absence of oxidation of alkene at 50°C in the absence of a compound of formula (I) according to the invention

### Materials and Methods

To an oven dried Schlenk flask was added EtOH/H₂O (96/4) (3mL), tert-butylstyrene (52 mg, 60 µL, 0.325 mmol, 1 equiv.) and dodecane (0.081 mmol, 0.25 equiv.) The Schlenk was heated up to 50°C and PhSiH₃ (102 mg, 117 µL, 0.975 mmol, 3 equiv.) was added. A balloon filled with air was added onto the Schlenk and the reaction mixture was stirred for 24 hours. Conversion and estimated yield were monitored by GC using dodecane as the internal standard. The reaction mechanism was as follows:

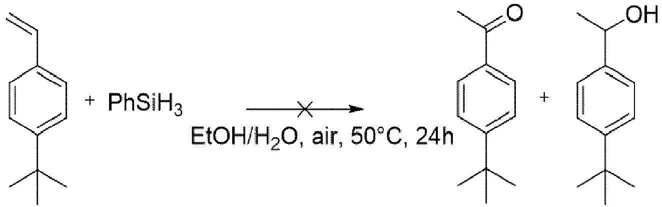

### Results

After 24 hours of reaction, no product, nor conversion of both alkene and PhSiH₃ (less than 5%) were detected clearly showing that the catalyst is responsible for the fast degradation of PhSiH₃ as well as the formation of oxidated product at this temperature.

### Example 10: Absence of oxidation of alkene at 20°C in the absence of a compound of formula (I) according to the invention

### Materials and Methods

To and oven dried Schlenk flask was added EtOH/H₂O (96/4) (3mL), tert-butylstyrene (52 mg, 60 µL, 0.325 mmol, 1 equiv.), PhSiH₃ (102 mg, 117 µL, 0.975 mmol, 3 equiv.) and dodecane (0.081 mmol, 0.25 equiv.). A balloon filled with air was added onto the Schlenk and the reaction mixture was stirred at 20°C for 24 hours. No traces of starting material conversion nor product formation were detected. The reaction mechanism was as follows:

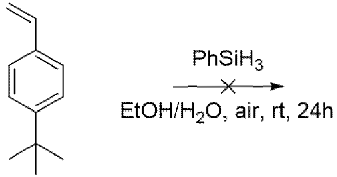

### Results

After 24 hours of reaction, no traces of starting material conversion nor product formation were detected.

### Example 11: Use of various solvents during the process according to the invention

### Materials and Methods

To an oven dried Schlenk flask was added Fe-C3 (2.5 mol %), the solvent (3mL), tert-butylstyrene and Et₃SiH (2 equiv.). A round-bottom flask filled with pure oxygen was added onto the Schlenk flask and the reaction mixture was stirred at room temperature for 24 hours.

The reaction mechanism was as follows:

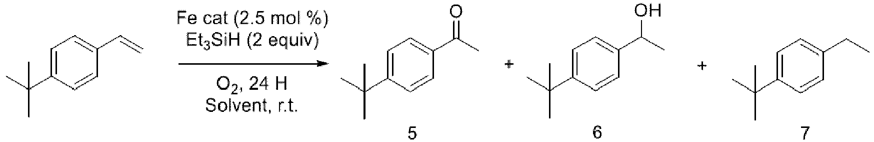

The Fe catalyst was compound Fe-C3 (synthesized in Example 3 of the present application). "rt" = room temperature.

### Results

The results are presented in the following table:

**Table 4**

| Solvent | Conversion of tert-butylstyrene (% w/w relative to the total weight of the tert-butylstyrene)^{∗} | Compound of formula (5) produced (% w/w relative to the total weight of the products)* | Compound of formula (6) produced (% w/w relative to the total weight of the products)^{∗} | Compound of formula (7) produced (% w/w relative to the total weight of the products)* |
|---|---|---|---|---|
| MeOH | 57 | 49 | 5 | 3 |
| EtOH/H₂O (96/4) | 100 | 98 | 2 | 0 |
| EtOH/H₂O (98/2) | 86 | 82 | 4 | 0 |

| | | | | |
|---|---|---|---|---|
| *Calculated by GC-MS | | | | |

The results thus obtain show that the use of a compound of formula (I) according to the invention, such as compound Fe-C3, results in a good conversion of tert-butylstyrene to ketone as the main product, in various solvents. This conversion is even better when the solvent is ethanol comprising 4% v/v of water.

Therefore, the use of a compound of formula (I) according to the invention allows a highly selective oxidation of an alkene into a ketone, in various solvents.

### Example 12: Use of air or O₂ during the process according to the invention

### Materials and Methods

To an oven dried schlenk flask was added Fe-C3, EtOH/H₂O (96/4) (3mL), tert-butylstyrene (52 mg, 60 µL, 0.325 mmol, 1 equiv.), Et₃SiH, and dodecane (0.081 mmol, 0.25 equiv.). A ballon filled with pure oxygen or air was added onto the schlenk and the reaction mixture was stirred at room temperature for a given time (24 hours or 48 hours).

The reaction mechanism was as follows:

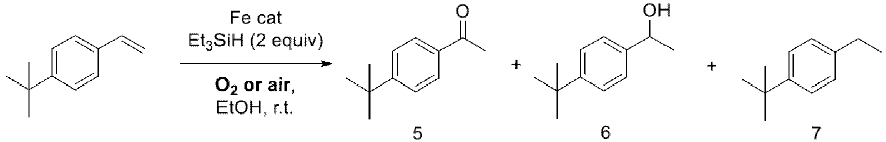

The Fe catalyst was compound Fe-C3 (synthesized in Example 3 of the present application). "rt" = room temperature.

### Results

The results are presented in the following table:

**Table 5**

| Cat.load.* | O₂ or air | Time (hours) | Conv.* | (5)* | (6)* | (7)* |
|---|---|---|---|---|---|---|
| 2.5 | O₂ | 24 | 100 | 98 | 0 | 2 |
| 2.5 | Air | 24 | 100 | 87 | 8 | 5 |
| 2.5 | Air | 48 | 100 | 95 | 0 | 5 |
| 5 | Air | 24 | 100 | 90 | 7 | 3 |
| 5 | Air | 48 | 100 | 97 | 0 | 3 |

| | | | | | | |
|---|---|---|---|---|---|---|
| * Cat.load. = catalyst loading (mol %). Conv. = Conversion of tert-butylstyrene (% w/w relative to the total weight of the tert-butylstyrene, calculated by GC-MS). (5) = Compound of formula (5) produced (% w/w relative to the total weight of the products, calculated by GC-MS). (6) = Compound of formula (6) produced (% w/w relative to the total weight of the products, calculated by GC-MS). (7) = Compound of formula (7) produced (% w/w relative to the total weight of the products, calculated by GC-MS). | | | | | | |

Therefore, the use of a compound of formula (I) according to the invention, such as compound Fe-C3, results in a good conversion of tert-butylstyrene to ketone as the main product, in the presence of air or pure oxygen. This conversion is even better in the presence of pure oxygen.

Therefore, the use of a compound of formula (I) according to the invention allows a highly selective oxidation of an alkene into a ketone, in the presence of air or pure oxygen.

### Example 13: Determination of turn-over number (=TON)

### Materials and Methods

To an oven dried Schlenk flask was added mL of a saturated diiron catalyst Fe-4 (synthesized in Example 5 of the present application), EtOH/H₂O (96/4), tert-butylstyrene (1 equiv.), PhSiH₃ (3 equiv.). A balloon filled with air was added onto the Schlenk and the reaction mixture was stirred at 20°C or at 50°C for 1 week. Conversion and estimated yield were monitored by GC-MS. Small aliquots of the solution were taken every 24h for 120h. No conversion of alkene was detected after 48h of reaction. The final TON calculated is an average value of 4 different estimated yield taken after 48, 72, 96 and 120h of reaction respectively.

### Results

The results are presented in the table below:

**Table 6:**

| Temperature | TON |
|---|---|
| 20°C | 40 000 |
| 50°C | 200 000 |

## Claims

1. Use of a compound of formula (I) as a catalyst for the oxidation of an alkene into a ketone, wherein formula (I) is as follows: 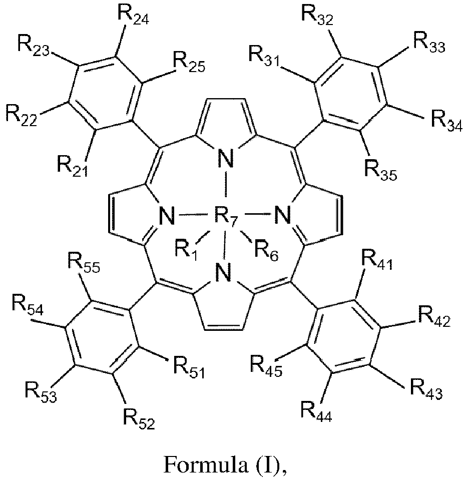 wherein R₆, which may be present or absent, is an optional group -Z, wherein group -Z is as follows: 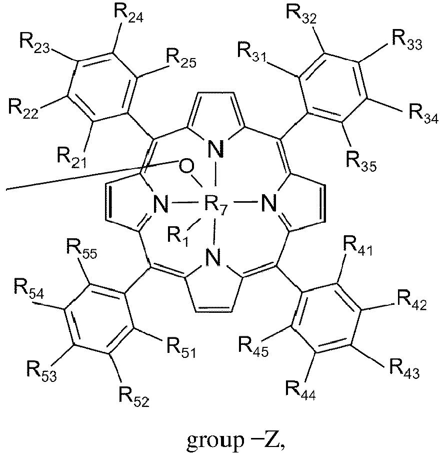 wherein, for formula (I) and for group -Z:
- R₁, which may be present or absent, is an optional anion selected from the group consisting of: Cl⁻, Br⁻, HO⁻ and CH₃COO⁻;
- R₂₁, R₂₂, R₂₃, R₂₄, R₂₅, R₃₁, R₃₂, R₃₃, R₃₄, R₃₅, R₄₁, R₄₂, R₄₃, R₄₄, R₄₅, R₅₁, R₅₂, R₅₃, R₅₄ and R₅₅ are selected, independently from each other, from the group consisting of: -H, -F, -CO₂H, -SO₃H and -H₂PO₃; and
- R₇ is selected from the group consisting of: -Fe and -Ni,
wherein, in formula (I), when R₇ is -Ni, R₆ is always absent and R₁ is present if R₇ is -Ni(III) and R₁ is absent if R₇ is -Ni(II).

2. The use according to claim 1, wherein the compound of formula (I) has the following formula (Ia3): 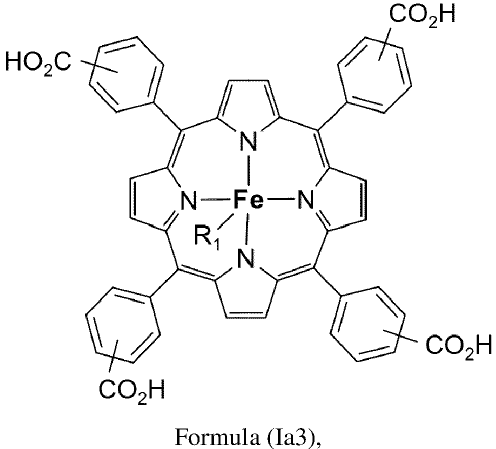 wherein R₁ is as defined in claim 1,
preferably, the compound of formula (I) has the following formula (Ib): 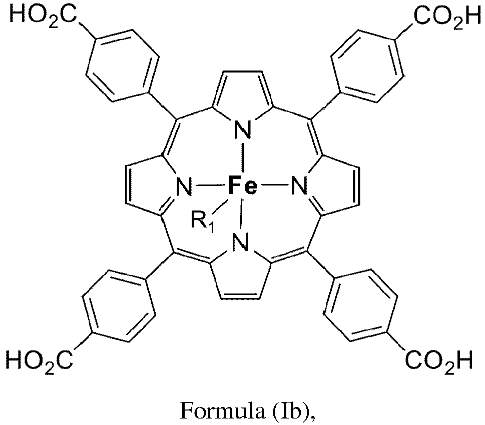 wherein R₁ is as defined in claim 1.

3. The use according to claim **1,** wherein the compound of formula (I) has the following formula (Ie):

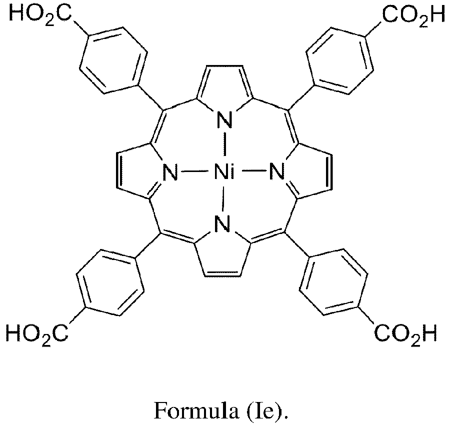

4. The use according to claim **1,** wherein the compound of formula (I) has the following formula (If):

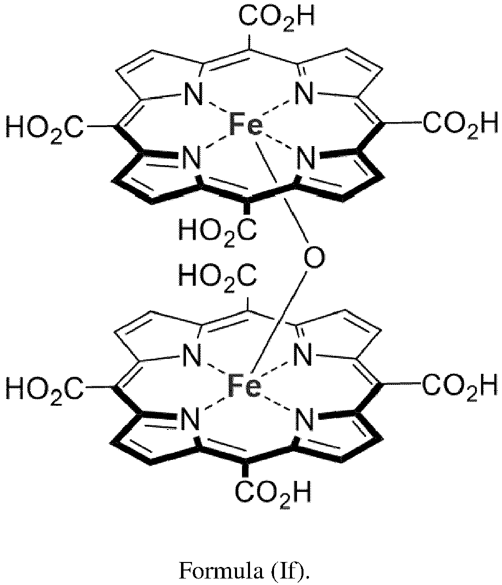

5. The use according to any one of claims **1** to **4,** wherein R₁ is Cl⁻.

6. The use according to any one of claims **1** to **5,** wherein the alkene is an aromatic alkene, preferably said alkene is selected from the group consisting of styrene, *tert-*butylstyrene, 3-methyl-1*H*-indole, 4-methoxystyrene, 4-chlorostyrene, 4-bromostyrene, 4-trifluoromethyl-styrene, 4-methoxycarbonylstyrene, 4-carboxystyrene, 2,4-dimethylstyrene, 4-vinylpyridine and mixtures thereof, more preferably said alkene is styrene.

7. A process of oxidation of an alkene into a ketone comprising a step (a) of reacting said alkene with an oxidant in the presence of a catalyst, wherein said catalyst is a compound of formula (I) as defined in any one of claims **1** to **5.**

8. The process according to claim **7,** wherein step (a) is carried out at a temperature ranging from 0°C to 100°C, preferably at a temperature ranging from 0°C to 50°C, more preferably at a temperature ranging from 15°C to 30°C, even more preferably at a temperature ranging from 20°C to 25°C, better at a temperature of about 20°C.

9. The process according to claim **7** or **8,** wherein step (a) is carried out in the presence of at least one compound selected from the group consisting of: 1,1,3,3-tetramethyldisiloxane and compounds of formula R_{4-y}SiH_{y} (II), wherein y is an integer ranging from 1 to 3 and R is selected from the group consisting of a C₁-C₆ alkyl and an aryl,
preferably step (a) is carried out in the presence of at least one compound selected from the group consisting of: 1,1,3,3-tetramethyldisiloxane, Et₃SiH, Ph₃SiH, Ph₂SiH₂ and PhSiH₃,
more preferably step (a) is carried out in the presence of at least one compound selected from the group consisting of: Et₃SiH, Ph₃SiH, Ph₂SiH₂ and PhSiH₃.

10. The process according to any one of claims **7** to **9,** wherein step (a) is carried out in the presence of a polymethylhydrosiloxane, preferably a polymethylhydrosiloxane having a weight average molar mass ranging from 5,000 g.mol⁻¹ to 400,000 g.mol⁻¹.

11. The process according to any one of claims **7** to **10,** wherein the alkene is an aromatic alkene, preferably said alkene is selected from the group consisting of styrene, tert-butylstyrene, 3-methyl-1*H*-indole, 4-methoxystyrene, 4-chlorostyrene, 4-bromostyrene, 4-trifluoromethyl-styrene, 4-methoxycarbonylstyrene, 4-carboxystyrene, 2,4-dimethylstyrene, 4-vinylpyridine and mixtures thereof, more preferably said alkene is styrene.

12. The process according to any one of claims **7** to **11,** wherein the oxidant is selected from the group consisting of O₂, H₂O₂, and potassium peroxymonosulfate, preferably the oxidant is O₂.

13. The process according to any one of claims **7** to **12,** wherein the alkene and the catalyst are in the same phase or in different phases during step (a), preferably the alkene and the catalyst are in the same phase during step (a).

14. The process according to any one of claims **7** to **13,** wherein step (a) is carried out in a homogeneous liquid medium comprising a solvent comprising at least 1% v/v of water, preferably step (a) is carried out in a homogeneous liquid medium comprising an alcohol-based solvent comprising at least 1% v/v of water, more preferably step (a) is carried out in a homogeneous liquid medium comprising an alcohol-based solvent comprising from 2% v/v to 10% v/v of water, even more preferably step (a) is carried out in a homogeneous liquid medium comprising ethanol as solvent, said ethanol comprising about 4% v/v of water.

15. The process according to any one of claims **7** to **14,** wherein the step (a) is followed by a step (b) of recovering the compound of formula (I), preferably the step (a) is followed by a step (b) of recovering the compound of formula (I) by acid/base precipitation.
